# EUROPEAN PATENT APPLICATION

(11) **EP 4 803 196 A1**
(43) Date of publication of application: **09.09.2026**
(21) Application number: 24896560.0
(22) Date of filing: 27.11.2024
(51) Int. Cl.: B01L 3/00, G01N 33/543, G01N 33/58

(54) **MAGNETIC BEAD MONODISPERSION CHIP AND OPERATION METHOD**

(30) Priority: 01.12.2023 CN 202311663599
(71) Applicant: Shenzhen Gate Biotech Co. Ltd, Guangdong 518057 (CN)
(72) Inventor: YANG, Haibo, Shenzhen, Guangdong 518057 (CN); DAI, Chongchong, Shenzhen, Guangdong 518057 (CN); TAN, Haotian, Shenzhen, Guangdong 518057 (CN); ZHANG, Qingyang, Shenzhen, Guangdong 518057 (CN)
(74) Representative: KASTEL Patentanwälte PartG mbB
(86) International application number: PCT/CN2024/134796
(87) International publication number: WO 2025/113474

(57) **Abstract**

A magnetic bead monodispersion chip and an operation method. The magnetic bead monodispersion chip comprises a droplet generation array (103) located in a housing. The droplet generation array (103) is located between a droplet storage cavity (102) and a sample inlet. The droplet generation array (103) comprises a plurality of micro-droplet generation channels. Each channel comprises a narrow channel region (I) and a bell-mouth region (II) connected to the narrow channel region (I). A channel bottom surface (103d) is higher than the bottom surface of the droplet storage cavity (102). The narrow channel region (I) is communicated with the sample inlet, and the bell-mouth region (II) is communicated with the droplet storage cavity (102). The channel has a first height. The ratio of the first height to the particle size of a magnetic bead is 1.5 to 3. The bell-mouth region (II) has a first width adjacent to the narrow channel region (I) and a second width adjacent to the droplet storage cavity (102), the second width being greater than the first width. By dispersing magnetic beads by means of micro-droplets, monodispersion of the magnetic beads can be achieved.

## Description

This application claims priority to Chinese Patent Application No. 202311663599.2, titled "MAGNETIC BEAD MONODISPERSION CHIP AND OPERATION METHOD", filed on December 01, 2023 with the China National Intellectual Property Administration.

### FIELD

The present disclosure relates to the field of biological analysis technology, and in particular to a magnetic bead monodispersion chip and an operation method.

### BACKGROUND

Digital enzyme-linked immunosorbent assay (digital ELISA) is configured to detect and measure the concentration of specific biomolecules (e.g., IL-6 antigen, or the like) in biological fluids. Compared with traditional ELISA, the digital ELISA has higher sensitivity and accuracy, enabling the detection and quantification of molecules at extremely low concentrations. Single-molecule immunoassay technology based on the digital ELISA can generally be divided into three parts: reagent reaction, magnetic bead monodispersion, and fluorescence detection.

In current commercial digital ELISA methods, there are two types of magnetic bead monodispersion: magnetic bead well loading method and magnetic bead planar spreading method. The magnetic bead well loading method utilizes gravity to capture magnetic beads in femtoliter-sized microwells. However, at the micro-scale, the effect of gravity is not significant, resulting in low and unstable capture efficiency of the magnetic bead well loading and significant loss of magnetic beads. The magnetic bead planar spreading method utilizes gravity sedimentation to planarly disperse magnetic beads on a smooth glass surface. Due to interactions such as non-specific adsorption forces and van der Waals forces between individual magnetic beads, unavoidable agglomeration of magnetic bead occurs after planar spreading, resulting in poor and unstable magnetic bead monodispersion.

In summary, the current effect of magnetic bead monodispersion is poor, and existing methods for magnetic bead monodispersion need further improvement.

### SUMMARY

The technical problem solved by the present disclosure is to provide a magnetic bead monodispersion chip and an operation method, so as to improve the magnetic bead monodispersion performance.

To solve the above technical problem, a magnetic bead monodispersion chip is provided according to a technical solution of the present disclosure. The magnetic bead monodispersion chip includes: a housing, where the housing is provided with a sample inlet and a sample outlet each communicating an interior and an exterior of the housing, the sample inlet is for injecting a magnetic bead suspension into the housing, the magnetic bead suspension includes multiple to-be-processed magnetic beads, and the to-be-processed magnetic beads have a first particle size; a droplet storage chamber located within the housing, where the droplet storage chamber has a storage chamber bottom surface and a storage chamber sidewall perpendicular to the storage chamber bottom surface; a droplet generation array located within the housing, where the droplet generation array is further located between the droplet storage chamber and the sample inlet, the droplet generation array includes multiple micro-droplet generation channels, each of the multiple micro-droplet generation channels includes a narrow channel region and a flared region communicating with the narrow channel region, the micro-droplet generation channel has a channel bottom surface and a channel sidewall perpendicular to the channel bottom surface, the channel bottom surface is higher than the storage chamber bottom surface, the narrow channel region is communicated with the sample inlet, the flared region is communicated with the droplet storage chamber, in a first direction perpendicular to the channel bottom surface, the micro-droplet generation channel has a first height, a ratio of the first height of the micro-droplet generation channel to the first particle size ranges from 1.5 to 3, and in a second direction parallel to the channel bottom surface and perpendicular to an extension direction of the micro-droplet generation channel, the flared region has a first width adjacent to the narrow channel region and a second width adjacent to the droplet storage chamber, and the second width is greater than the first width; and an oil storage chamber located within the housing, where the oil storage chamber is further located between the droplet storage chamber and the sample outlet, is communicated with the sample outlet, and is for storing an oil phase reagent flowing out of the droplet storage chamber.

Optionally, a structure of the magnetic bead monodispersion chip further includes: a droplet filtering structure located between the droplet storage chamber and the oil storage chamber, the droplet filtering structure has multiple gaps communicating the droplet storage chamber and the oil storage chamber, a width of each of the gaps is smaller than a target droplet size, and the width of each of the gaps ranges from 10 µm to 40 µm.

Optionally, each of the micro-droplet generation channels further includes an inlet region, two ends of the inlet region are communicated with the narrow channel region and the sample inlet, respectively; the inlet region has a third width adjacent to the narrow channel region and a fourth width adjacent to the sample inlet, and the fourth width is greater than the third width.

Optionally, the droplet generation array is in a shape of a sector ring, and the multiple micro-droplet generation channels are arranged along a circumferential direction.

Optionally, a number of the multiple micro-droplet generation channels is one of 8, 16, 24, 32, and 48.

Optionally, a structure of the magnetic bead monodispersion chip further satisfies that: the first height ranges from 5 µm to 20 µm; a ratio of a width of the narrow channel region in the second direction to the first particle size ranges from 2 to 10; the width of the narrow channel region in the second direction ranges from 10 µm to 25 µm; an angle between the channel sidewall of the flared region and the channel sidewall of the narrow channel region ranges from 10 degrees to 20 degrees; a length of the flared region in the extension direction of the micro-droplet generation channel ranges from 50µm to 500µm; and a ratio of a height difference between the channel bottom surface and the storage chamber bottom surface to the first height ranges from 4 to 15.

Optionally, the housing includes an upper housing and a lower housing, the upper housing and the lower housing are sealed at edges, and the sample inlet and the sample outlet are located on the upper housing; the magnetic bead monodispersion chip further includes: multiple support pillars located between the storage chamber bottom surface and the upper housing; the multiple support pillars are further located between a bottom surface of the oil storage chamber and the upper housing; a radius of each of the support pillars ranges from 100µm to 500µm; the multiple support pillars are arranged in an array in a direction parallel to the storage chamber bottom surface, a spacing distance between two adjacent support pillars ranges from 1mm to 2.5mm; a thickness of the upper housing ranges from 0.5mm to 2mm; and a thickness of the lower housing ranges from 0.5mm to 2mm.

Optionally, a material of the magnetic bead monodispersion chip includes one or more selected from the group consisting of glass, silicon wafer, and polymer material; the polymer material includes one or more selected from the group consisting of polydimethylsiloxane, polyurethane, epoxy resin, polymethyl methacrylate, polycarbonate, cyclic olefin copolymer, polystyrene, polyethylene, and fluoroplastic.

Accordingly, an operation method for magnetic bead monodispersion is further provided according to a technical solution of the present disclosure. The operation method includes: providing a magnetic bead monodispersion chip, where the magnetic bead monodispersion chip includes: a housing, where the housing is provided with a sample inlet and a sample outlet each communicating an interior and an exterior of the housing, the sample inlet is for injecting a magnetic bead suspension into the housing, the magnetic bead suspension includes multiple to-be-processed magnetic beads, and the to-be-processed magnetic beads have a first particle size; a droplet storage chamber located within the housing, where the droplet storage chamber has a storage chamber bottom surface and a storage chamber sidewall perpendicular to the storage chamber bottom surface; a droplet generation array located within the housing, where the droplet generation array is further located between the droplet storage chamber and the sample inlet, the droplet generation array includes multiple micro-droplet generation channels, each of the multiple micro-droplet generation channels includes a narrow channel region and a flared region communicating with the narrow channel region, the micro-droplet generation channel has a channel bottom surface and a channel sidewall perpendicular to the channel bottom surface, the channel bottom surface is higher than the storage chamber bottom surface, the narrow channel region is communicated with the sample inlet, the flared region is communicated with the droplet storage chamber, in a first direction perpendicular to the channel bottom surface, the micro-droplet generation channel has a first height, and a ratio of the first height of the micro-droplet generation channel to the first particle size ranges from 1.5 to 3, and in a second direction parallel to the channel bottom surface and perpendicular to an extension direction of the micro-droplet generation channel, the flared region has a first width adjacent to the narrow channel region and a second width adjacent to the droplet storage chamber, and the second width is greater than the first width; and an oil storage chamber located within the housing, where the oil storage chamber is further located between the droplet storage chamber and the sample outlet, is communicated with the sample outlet, and is for storing an oil phase reagent flowing out of the droplet storage chamber; providing the magnetic bead suspension; injecting the oil phase reagent into the magnetic bead monodispersion chip through the sample inlet until no gas remains inside the magnetic bead monodispersion chip; and after injecting the oil phase reagent, injecting the magnetic bead suspension into the magnetic bead monodispersion chip.

Optionally, the method of injecting the magnetic bead suspension into the magnetic bead monodispersion chip includes: using a segmented aspiration method to pre-encapsulate the magnetic bead suspension to be processed into a flexible tube, where the segmented aspiration method includes causing the flexible tube to aspirate at least a first segment of the oil phase reagent, and after aspirating the first segment of the oil phase reagent, aspirating the magnetic bead suspension to be processed; hermetically connecting an aspiration port end of the flexible tube to the sample inlet of the magnetic bead monodispersion chip, ensuring that there are no air bubbles at a connection; and after hermetically connecting, injecting the magnetic bead suspension to be processed and the first segment of the oil phase reagent from the flexible tube into the magnetic bead monodispersion chip.

Optionally, the segmented aspiration method further includes: after aspirating the magnetic bead suspension to be processed, aspirating a second segment of the oil phase reagent.

Optionally, the method of obtaining the magnetic bead suspension includes: obtaining a target magnetic bead concentration according to a target droplet size; producing the magnetic bead suspension having the target magnetic bead concentration.

Optionally, the method of obtaining the target magnetic bead concentration according to the target droplet size includes: obtaining a target droplet size D1; according to the target droplet size D1, obtaining a boundary particle size D2 of the magnetic bead suspension to be processed, where D2 > D1/90%; according to a relationship between a magnetic bead concentration C in the magnetic bead suspension and the boundary particle size D2, which is $\frac{1}{C} = \frac{4}{3} π {\left(\frac{D 2}{2}\right)}^{3}$, and based on the boundary particle size D2, obtaining the target magnetic bead concentration C of the magnetic bead suspension.

Optionally, the method of producing the magnetic bead suspension having the target magnetic bead concentration further includes: obtaining an initial suspension, where the initial suspension includes the multiple to-be-processed magnetic beads; adding a surfactant to the initial suspension, and performing a resuspension treatment on the initial suspension to obtain the magnetic bead suspension, where the resuspension treatment method includes one or more of vortexing, stirring, and shaking; and the surfactant includes one or more selected from the group consisting of Tween 20, SDS, Span 80, Triton, and EM90.

Optionally, the structure of the magnetic bead monodispersion chip satisfies that: the first height ranges from 5 µm to 20 µm; a ratio of a width of the narrow channel region in the second direction to the first particle size ranges from 2 to 10; the width of the narrow channel region in the second direction ranges from 10 µm to 25 µm; an angle between the channel sidewall of the flared region and the channel sidewall of the narrow channel region ranges from 10 degrees to 20 degrees; a length of the flared region in the extension direction of the micro-droplet generation channel ranges from 50µm to 500µm; and a ratio of a height difference between the channel bottom surface and the storage chamber bottom surface to the first height ranges from 4 to 15.

Optionally, the method of obtaining the magnetic bead monodispersion chip includes: establishing a model of droplet size and setting parameters of the magnetic bead monodispersion chip, where the setting parameters of the magnetic bead monodispersion chip includes one or more of the first height, the width of the narrow channel region in the second direction, the first width and the second width of the flared region, an angle of inclination of a sidewall of the flared region relative to the sidewall of the narrow channel region, a length of the flared region in the extension direction of the micro-droplet generation channel, and a height difference between the channel bottom surface and the storage chamber bottom surface; obtaining values of the setting parameters of the magnetic bead monodispersion chip according to the target droplet size; and producing the magnetic bead monodispersion chip according to the values of the setting parameters of the magnetic bead monodispersion chip.

Optionally, the structure of the magnetic bead monodispersion chip further includes: a droplet filtering structure located between the droplet storage chamber and the oil storage chamber, the droplet filtering structure has multiple gaps communicating the droplet storage chamber and the oil storage chamber, a width of each of the gaps is smaller than a target droplet size, and the width of each of the gaps ranges from 10 µm to 40 µm.

Optionally, the droplet generation array is in a shape of a sector ring, and the multiple micro-droplet generation channels are arranged along a circumferential direction.

Optionally, the housing includes an upper housing and a lower housing, the upper housing and the lower housing are sealed at edges, and the sample inlet and the sample outlet are located on the upper housing; the magnetic bead monodispersion chip further includes: multiple support pillars located between the storage chamber bottom surface and the upper housing; the multiple support pillars are further located between a bottom surface of the oil storage chamber and the upper housing; a radius of each of the support pillars ranges from 100µm to 500µm; the multiple support pillars are arranged in an array in a direction parallel to the storage chamber bottom surface, a spacing distance between two adjacent support pillars ranges from 1mm to 2.5mm; a thickness of the upper housing ranges from 0.5mm to 2mm; and a thickness of the lower housing ranges from 0.5mm to 2mm.

Compared with the conventional technology, the technical solution of embodiments of the present disclosure has the following beneficial effects. In the magnetic bead monodispersion chip provided by the technical solution of the present disclosure, the droplet generation array includes the multiple micro-droplet generation channels, and the channel bottom surface is higher than the storage chamber bottom surface, such that a step between the channel and the droplet storage chamber is formed. After the magnetic bead suspension enters the droplet generation array, droplets are formed at the step due to a sudden change in surface tension. In addition, the channel has a first height, and the ratio of the first height to the first particle size of the to-be-processed magnetic beads is limited, which facilitates enabling only one single magnetic bead to pass through a single channel at a time, thereby facilitating encapsulation of a single magnetic bead in a single droplet to form a micro-droplet. The form of dispersing magnetic beads via micro-droplets reduces the probability of magnetic bead re-agglomeration after dispersion, which is conductive to achieving monodispersion of magnetic beads.

Furthermore, the droplet generation array is in a shape of a sector ring, and the sector ring structure makes generated micro-droplets less prone to accumulation, thereby facilitating improving the uniformity of sizes of the micro-droplets.

In the operation method for a magnetic bead monodispersion chip provided by the technical solution of the present disclosure, the droplet generation array includes multiple micro-droplet generation channels, and the channel bottom surface is higher than the storage chamber bottom surface to form a step between the channel and the droplet storage chamber. After the magnetic bead suspension enters the droplet generation array, droplets are formed at the step due to a sudden change in surface tension. In addition, the channel has a first height, and the ratio of the first height to the first particle size of the to-be-processed magnetic beads is limited, which facilitates enabling only one single magnetic bead to pass through a single channel at a time, thereby facilitating encapsulation of a single magnetic bead in a single droplet to form a micro-droplet. The form of dispersing magnetic beads via micro-droplets reduces the probability of magnetic bead re-agglomeration after dispersion, which is conductive to achieving monodispersion of magnetic beads.

Furthermore, obtaining a target magnetic bead concentration according to a target droplet size enables obtaining a magnetic bead concentration that facilitates magnetic beads monodispersion, thereby increasing the proportion of single-bead encapsulation ultimately obtained.

Furthermore, the segmented aspiration method facilitates improving the utilization rate of magnetic beads, achieving a magnetic bead utilization rate close to 100%.

### BRIEF DESCRIPTION OF THE DRAWINGS

Hereinafter drawings required for describing embodiments or the conventional technology will be briefly introduced, in order to more clearly illustrate technical solutions in the embodiments of the present disclosure or the conventional technology. Apparently, the drawings in the following description are only some embodiments of the present disclosure, and for those skilled in the art, other drawings may be obtained based on these drawings without creative effort.
FIGs. 1 to 4 are schematic structural diagrams of a magnetic bead monodispersion chip according to an embodiment of the present disclosure.
FIGs. 5 to 9 are schematic flow diagrams of an operation method for a magnetic bead monodispersion chip according to an embodiment of the present disclosure.
FIG. 10 is a schematic diagram of a segmented aspiration method in an operation method for a magnetic bead monodispersion chip according to an embodiment of the present disclosure.
FIGs. 11 to 13 are schematic diagrams of magnetic bead droplets obtained by an operation method for a magnetic bead monodispersion chip according to an embodiment of the present disclosure.
FIG. 14 is a schematic structural diagram of a magnetic bead monodispersion chip according to another embodiment of the present disclosure.

### DETAILED DESCRIPTION OF EMBODIMENTS

As described in the background art, the existing effect of magnetic bead monodispersion is poor, and existing methods for magnetic bead monodispersion need further improvement.

To solve the above problem, in the operation method for a magnetic bead monodispersion chip provided by the technical solution of the present disclosure, the droplet generation array includes multiple micro-droplet generation channels, and the channel bottom surface is higher than the storage chamber bottom surface to form a step between the channel and the droplet storage chamber. After the magnetic bead suspension enters the droplet generation array, droplets are formed at the step due to a sudden change in surface tension. In addition, the channel has a first height, and the ratio of the first height to the first particle size of the to-be-processed magnetic beads is limited, which facilitates enabling only one single magnetic bead to pass through a single channel at a time, thereby facilitating encapsulation of a single magnetic bead in a single droplet to form a micro-droplet. The form of dispersing magnetic beads via micro-droplets reduces the probability of magnetic bead re-agglomeration after dispersion, which is conductive to achieving monodispersion of magnetic beads.

Hereinafter specific embodiments of the present disclosure will be described in detail with reference to the accompanying drawings, in order to make the above objectives, features, and beneficial effects of the present disclosure more apparent and understandable.

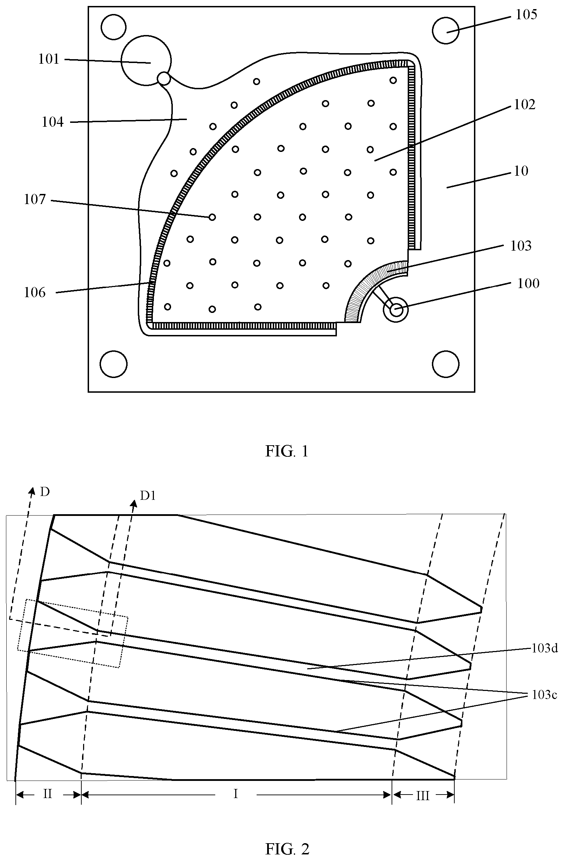

FIGs. 1 to 4 are schematic structural diagrams of a magnetic bead monodispersion chip according to an embodiment of the present disclosure.

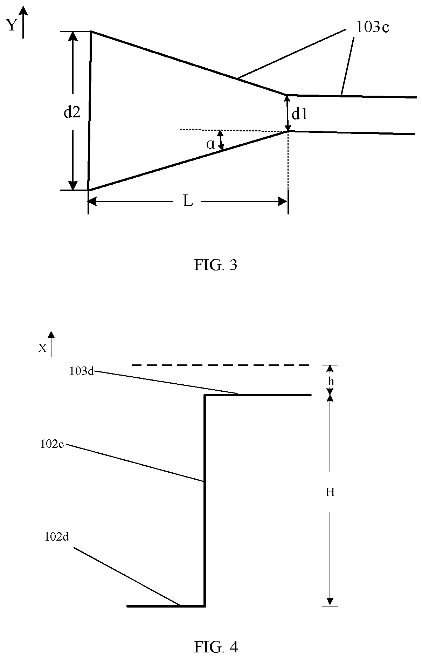

Reference is made to FIGs. 1 to 4. FIG. 1 is a top schematic structural view of the magnetic bead monodispersion chip with an upper housing omitted. FIG. 2 is a partially enlarged view of a droplet generation array in FIG. 1. FIG. 3 is a partially enlarged view of a dashed box region in FIG. 2. FIG. 4 is a schematic cross-sectional structural diagram along line D-D1 of the dashed box in FIG. 2. The magnetic bead monodispersion chip includes: a housing, where the housing is provided with a sample inlet (not shown in the figures) and a sample outlet (not shown in the figures) each communicating an interior and an exterior of the housing, the sample inlet is for injecting a magnetic bead suspension into the housing, the magnetic bead suspension includes multiple to-be-processed magnetic beads, and the to-be-processed magnetic beads have a first particle size; a droplet storage chamber 102 located within the housing, where the droplet storage chamber 102 has a storage chamber bottom surface 102d and a storage chamber sidewall 102c perpendicular to the storage chamber bottom surface 102d; a droplet generation array 103 located within the housing, where the droplet generation array 103 is further located between the droplet storage chamber 102 and the sample inlet, the droplet generation array 103 includes multiple micro-droplet generation channels, each of the channels includes a narrow channel region I and a flared region II communicating with the narrow channel region I, the channel has a channel bottom surface 103d and a channel sidewall 103c perpendicular to the channel bottom surface 103d, the channel bottom surface 103d is higher than the storage chamber bottom surface 102d, the narrow channel region I is communicated with the sample inlet, the flared region II is communicated with the droplet storage chamber 102, in a first direction X perpendicular to the channel bottom surface 103d, the channel has a first height h, a ratio of the first height h of the channel to the first particle size ranges from 1.5 to 3, and in a second direction Y parallel to the channel bottom surface 103d and perpendicular to an extension direction of the channel, the flared region II has a first width d1 adjacent to the narrow channel region I and a second width d2 adjacent to the droplet storage chamber 102, and the second width d2 is greater than the first width d1; an oil storage chamber 104 located within the housing, where the oil storage chamber 104 is further located between the droplet storage chamber 102 and the sample outlet, is communicated with the sample outlet, and is for storing an oil phase reagent flowing out of the droplet storage chamber 102.

Herein the droplet generation array 103 includes multiple micro-droplet generation channels. The channel bottom surface 103d is higher than the storage chamber bottom surface 102d to form a step between the channels and the droplet storage chamber 102. After the magnetic bead suspension enters the droplet generation array 103, droplets are generated at the step due to a sudden change in surface tension. The shape of the flared region II is conductive to improving monodispersity among droplets. In addition, the channel has a first height h, and the ratio of the first height h to the first particle size of the to-be-processed magnetic bead is limited, which facilitates enabling only one single magnetic bead to pass through a single channel at a time, thereby facilitating encapsulation of a single magnetic bead in a single droplet to form a micro-droplet. The form of dispersing magnetic beads via micro-droplets reduces the probability of magnetic bead re-agglomeration after dispersion, which is conductive to achieving monodispersion of magnetic beads.

In an embodiment, the housing includes an upper housing (not shown in the figures) and a lower housing 10. The upper housing and the lower housing 10 are sealed at edges. The sample inlet and the sample outlet are located on the upper housing.

The packaging method for the magnetic bead monodispersion chip may be one or more of thermal bonding, adhesive bonding, plasma bonding, and anodic bonding. Specifically, FIG. 1 further shows positions of auxiliary sealing holes 105.

In an embodiment, the droplet generation array 103, the droplet storage chamber 102, and the oil storage chamber 104 are all disposed on the lower housing 10.

In an embodiment, the magnetic bead monodispersion chip further includes: a sample inlet chamber 100 located within the housing, where the sample inlet chamber 100 is located between the sample inlet and the droplet generation array 103, and is communicated with the sample inlet and the droplet generation array 103; an oil drainage chamber 101 located within the housing, where the oil drainage chamber 101 is located between the sample outlet and the oil storage chamber 104, and is communicated with the sample outlet and the oil storage chamber 104.

In an embodiment, the structure of the magnetic bead monodispersion chip further includes: a droplet filtering structure 106 located between the droplet storage chamber 102 and the oil storage chamber 104. The droplet filtering structure 106 has multiple gaps (not shown in the figures) communicating the droplet storage chamber 102 and the oil storage chamber 104. A width of each of the gaps is smaller than a target droplet size. The width of each of the gaps ranges from 10 µm to 40 µm. The purpose of making the width of each of the gaps smaller than the target droplet size is to retain all droplets meeting the size requirement within the droplet storage chamber 102.

In an embodiment, each of the channels further includes an inlet region III, two ends of the inlet region III are communicated with the narrow channel region I and the sample inlet, respectively. The inlet region III has a third width adjacent to the narrow channel region I and a fourth width adjacent to the sample inlet, and the fourth width is greater than the third width. The inlet region III has a flared structure, which facilitates sample introduction into the droplet generation array.

In an embodiment, the droplet generation array is in a shape of a sector ring, and the multiple channels are arranged along a circumferential direction. The sector ring structure makes generated micro-droplets less likely to accumulate, which is conductive to improving the uniformity of micro-droplet sizes.

In an embodiment, the number of the multiple channels is one of 8, 16, 24, 32, and 48.

In an embodiment, the structure of the magnetic bead monodispersion chip further satisfies that: the first height h ranges from 5µm to 20µm; a ratio of a width of the narrow channel region I in the second direction Y to the first particle size ranges from 2 to 10; the width of the narrow channel region I in the second direction Y ranges from 10µm to 25µm; an angle α between the channel sidewall 103c of the flared region II and the channel sidewall 103c of the narrow channel region I ranges from 10 degrees to 20 degrees; a length L of the flared region II in the extension direction of the channel ranges from 50µm to 500µm; a ratio of a height difference H between the channel bottom surface 103d and the storage chamber bottom surface 102d to the first height h ranges from 4 to 15. By adjusting the above setting parameters of the magnetic bead monodispersion chip, it is advantageous to obtain a reasonable droplet size.

In an embodiment, the magnetic bead monodispersion chip further includes: multiple support pillars 107 located between the storage chamber bottom surface 102d and the upper housing. The multiple support pillars 107 are used to prevent the structure of the magnetic bead monodispersion chip from collapsing.

In an embodiment, the multiple support pillars 107 are further located between a bottom surface of the oil storage chamber 104 and the upper housing. In another embodiment, the oil storage chamber is small enough that the multiple support pillars may not be disposed between the bottom surface of the oil storage chamber and the upper housing.

In an embodiment, a radius of each of the support pillars 107 ranges from 100µm to 500µm; the multiple support pillars 107 are arranged in an array in a direction parallel to the storage chamber bottom surface 102d, a spacing distance between two adjacent support pillars 107 ranges from 1mm to 2.5mm; a thickness of the upper housing ranges from 0.5mm to 2mm; and a thickness of the lower housing ranges from 0.5mm to 2mm.

In an embodiment, a material of the magnetic bead monodispersion chip includes one or more of glass, silicon wafer, and polymer material. The polymer material includes one or more of polydimethylsiloxane, polyurethane, epoxy resin, polymethyl methacrylate, polycarbonate, cyclic olefin copolymer, polystyrene, polyethylene, and fluoroplastic.

It should be noted that the shape of the droplet storage chamber 102 is not limited. In an embodiment, the droplet storage chamber 102 is sector-shaped. In another embodiment, the droplet storage chamber 102 may be rectangular or other shapes.

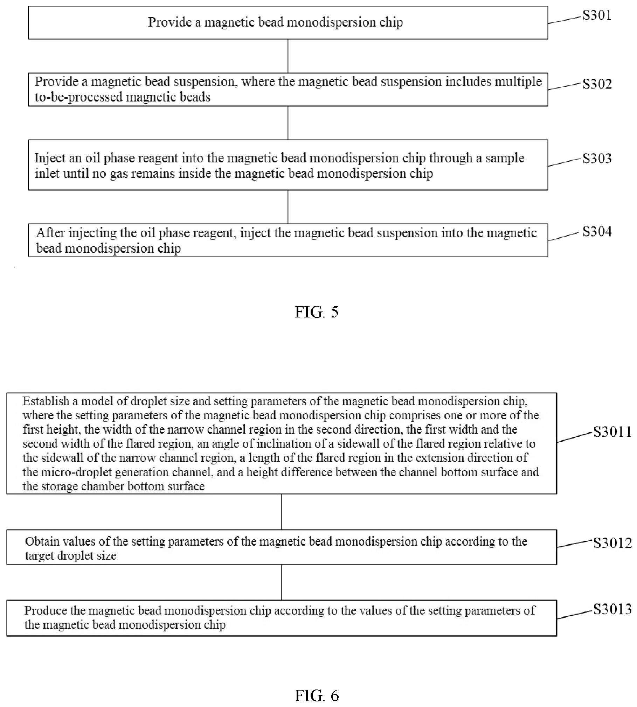

FIGs. 5 to 9 are schematic flow diagrams of an operation method for a magnetic bead monodispersion chip according to an embodiment of the present disclosure.

Accordingly, an operation method for the above magnetic bead monodispersion chip is further provided according to an embodiment of the present disclosure. Reference is made to FIG. 5, the method includes the following steps:
Step S301, providing a magnetic bead monodispersion chip;
Step S302, providing a magnetic bead suspension, where the magnetic bead suspension includes multiple to-be-processed magnetic beads;
Step S303, injecting an oil phase reagent into the magnetic bead monodispersion chip through the sample inlet until no gas remains inside the magnetic bead monodispersion chip;
Step S304, after injecting the oil phase reagent, injecting the magnetic bead suspension into the magnetic bead monodispersion chip.

A detailed description will be given below with reference to the accompanying drawings.

Please continue to refer to FIGs. 1 to 4, the magnetic bead monodispersion chip is provided.

The magnetic bead monodispersion chip includes: a housing, where the housing is provided with a sample inlet (not shown in the figures) and a sample outlet (not shown in the figures) each communicating an interior and an exterior of the housing, the sample inlet is for injecting the magnetic bead suspension into the housing, the magnetic bead suspension includes a plurality of to-be-processed magnetic beads, the to-be-processed magnetic beads have a first particle size; a droplet storage chamber 102 located within the housing, wherein the droplet storage chamber 102 has a storage chamber bottom surface 102d and a storage chamber sidewall 102c perpendicular to the storage chamber bottom surface 102d; a droplet generation array 103 located within the housing, where the droplet generation array 103 is further located between the droplet storage chamber 102 and the sample inlet, the droplet generation array 103 includes multiple micro-droplet generation channels, each of the channels includes a narrow channel region I and a flared region II communicating with the narrow channel region I, the channel has a channel bottom surface 103d and a channel sidewall 103c perpendicular to the channel bottom surface 103d, the channel bottom surface 103d is higher than the storage chamber bottom surface 102d, the narrow channel region I is communicated with the sample inlet, the flared region II is communicated with the droplet storage chamber 102, in a first direction X perpendicular to the channel bottom surface 103d, the channel has a first height h, and a ratio of the first height h of the channel to the first particle size ranges from 1.5 to 3, in a second direction Y parallel to the channel bottom surface 103d and perpendicular to an extension direction of the channel, the flared region II has a first width d1 adjacent to the narrow channel region I and a second width d2 adjacent to the droplet storage chamber 102, and the second width d2 is greater than the first width d1; an oil storage chamber 104 located within the housing, where the oil storage chamber 104 is further located between the droplet storage chamber 102 and the sample outlet, is communicated with the sample outlet, and is for storing an oil phase reagent flowing out of the droplet storage chamber 102.

In an embodiment, the first height h ranges from 5µm to 20µm. A ratio of a width of the narrow channel region I in the second direction Y to the first particle size ranges from 2 to 10. The width of the narrow channel region I in the second direction Y ranges from 10µm to 25µm. An angle α between the channel sidewall 103c of the flared region II and the channel sidewall 103c of the narrow channel region I ranges from 10 degrees to 20 degrees. A length L of the flared region II in the extension direction of the channel ranges from 50µm to 500µm. A ratio of a height difference H between the channel bottom surface 103d and the storage chamber bottom surface 102d to the first height h ranges from 4 to 15.

In an embodiment, the structure of the magnetic bead monodispersion chip further includes: a droplet filtering structure 106 located between the droplet storage chamber 102 and the oil storage chamber 104. The droplet filtering structure 106 has multiple gaps (not shown in the figures) communicating the droplet storage chamber 102 and the oil storage chamber 104, and a width of each of the gaps is smaller than a target droplet size. The width of each of the gaps ranges from 10µm to 40µm.

It should be noted that the gap has two opposite sidewalls perpendicular to the storage chamber bottom surface 102d, and the width refers to the distance between the two sidewalls.

In an embodiment, the droplet generation array is in a shape of a sector ring, and the multiple channels are arranged along a circumferential direction.

In an embodiment, the housing includes an upper housing (not shown in the figures) and a lower housing 10, the upper housing and the lower housing 10 are sealed at edges, and the sample inlet and the sample outlet are located on the upper housing.

In an embodiment, the magnetic bead monodispersion chip further includes: multiple support pillars 107 located between the storage chamber bottom surface 102d and the upper housing. The multiple support pillars 107 are further located between a bottom surface of the oil storage chamber 104 and the upper housing. A radius of each of the support pillars 107 ranges from 100µm to 500µm. The multiple support pillars 107 are arranged in an array in a direction parallel to the storage chamber bottom surface 102d. A spacing distance between two adjacent support pillars 107 ranges from 1mm to 2.5mm. A thickness of the upper housing ranges from 0.5 mm to 2 mm. A thickness of the lower housing ranges from 0.5mm to 2mm.

In an embodiment, reference is made to FIG. 6, the method of obtaining the magnetic bead monodispersion chip includes the following steps:
Step S3011, establishing a model of droplet size and setting parameters of the magnetic bead monodispersion chip, where the setting parameters of the magnetic bead monodispersion chip includes one or more of the first height, the width of the narrow channel region in the second direction, the first width and the second width of the flared region, an angle of inclination of a sidewall of the flared region relative to the sidewall of the narrow channel region, a length of the flared region in the extension direction of the micro-droplet generation channel, and a height difference between the channel bottom surface and the storage chamber bottom surface;
Step S3012, obtaining values of the setting parameters of the magnetic bead monodispersion chip according to the target droplet size; and
Step S3013, producing the magnetic bead monodispersion chip according to the values of the setting parameters of the magnetic bead monodispersion chip.

Reference is made to FIG. 5, the magnetic bead suspension is provided, and the magnetic bead suspension includes multiple to-be-processed magnetic beads.

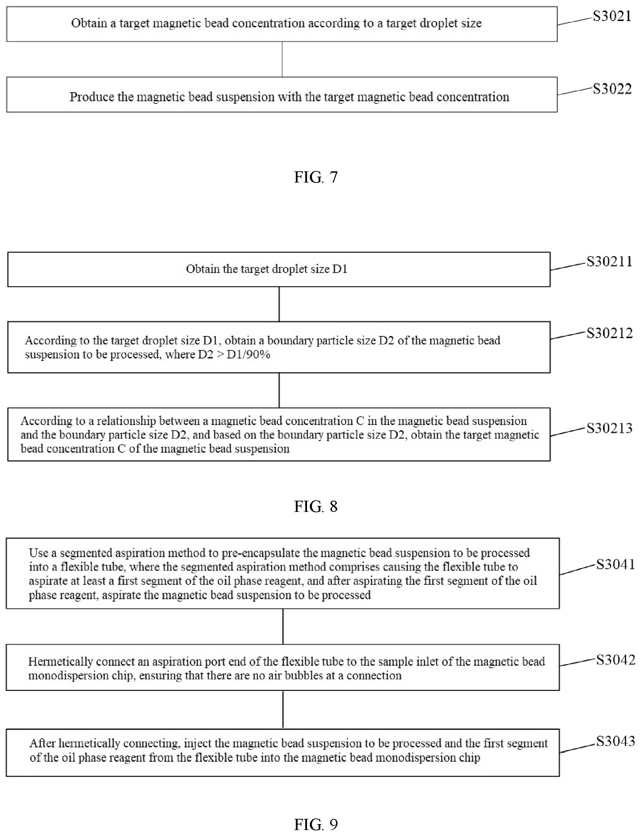

In an embodiment, reference is made to FIG. 7, the method of obtaining the magnetic bead suspension includes the following steps:
Step S3021, obtaining a target magnetic bead concentration according to a target droplet size;
Step S3022, producing the magnetic bead suspension with the target magnetic bead concentration.

In an embodiment, reference is made to FIG. 8, the method of obtaining the target magnetic bead concentration according to the target droplet size includes the following steps:
Step S30211, obtaining the target droplet size D1;
Step S30212, according to the target droplet size D1, obtaining a boundary particle size D2 of the magnetic bead suspension to be processed, where D2 > D1/90%;
Step S30213, according to a relationship between a magnetic bead concentration C in the magnetic bead suspension and the boundary particle size D2, which is $\frac{1}{C} = \frac{4}{3} π {\left(\frac{D 2}{2}\right)}^{3}$, and based on the boundary particle size D2, obtaining the target magnetic bead concentration C of the magnetic bead suspension.

Herein, based on practical experience, controlling the droplet size to be less than 90% of the boundary particle size is beneficial for achieving 100% proportion of single-bead encapsulation of magnetic beads, thereby obtaining a magnetic bead concentration favorable for achieving monodispersion of magnetic beads and increasing the proportion of single-bead encapsulation ultimately obtained. The proportion of single-bead encapsulation refers to the proportion of the number of magnetic beads that are encapsulated as "a single magnetic bead in a single droplet" relative to the total number of magnetic beads.

In an embodiment, the method of producing the magnetic bead suspension with the target magnetic bead concentration further includes: obtaining an initial suspension, where the initial suspension includes the multiple to-be-processed magnetic beads; adding a surfactant to the initial suspension, and performing a resuspension treatment on the initial suspension to obtain the suspension.

In an embodiment, the resuspension treatment method includes one or more of vortexing, stirring, and shaking; the surfactant includes one or more of Tween 20 (polysorbate 20), SDS (sodium dodecyl sulfate), Span 80 (span-80), Triton (span-80), and EM90.

In an embodiment, reference is made to FIG. 9, the method of injecting the magnetic bead suspension into the magnetic bead monodispersion chip includes the following steps:
Step S3041, using a segmented aspiration method to pre-encapsulate the magnetic bead suspension to be processed into a flexible tube, where the segmented aspiration method includes causing the flexible tube to aspirate at least a first segment of the oil phase reagent, and after aspirating the first segment of the oil phase reagent, aspirating the magnetic bead suspension to be processed;
Step S3042, hermetically connecting an aspiration port end of the flexible tube to the sample inlet of the magnetic bead monodispersion chip, ensuring that there are no air bubbles at a connection; and
Step S3043, after hermetically connecting, injecting the magnetic bead suspension to be processed and the first segment of the oil phase reagent from the flexible tube into the magnetic bead monodispersion chip.

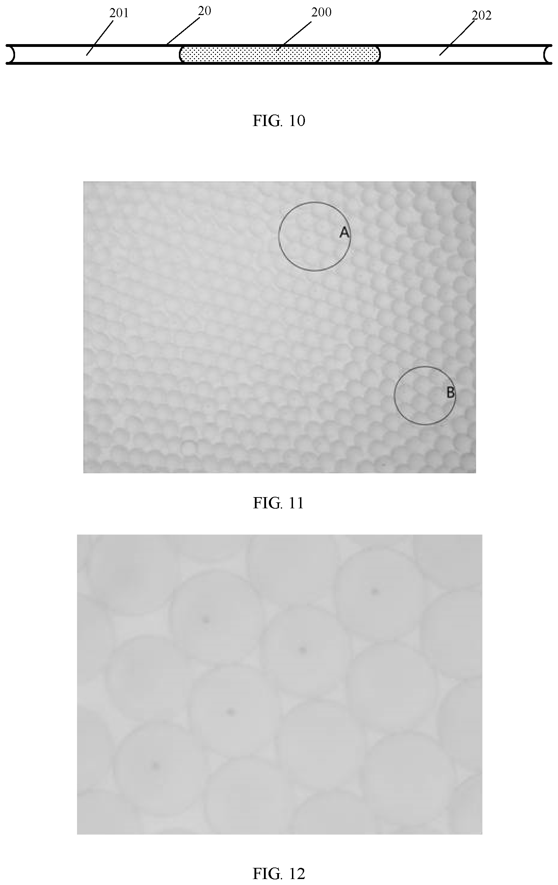

FIG. 10 is a schematic diagram of a segmented aspiration method in an operation method for a magnetic bead monodispersion chip according to an embodiment of the present disclosure.

Reference is made to FIGs. 9 and 10, a segmented aspiration method is used to pre-encapsulate a magnetic bead suspension 200 to be processed into a flexible tube 20. The segmented aspiration method includes causing the flexible tube 20 to aspirate at least a first segment of oil phase reagent 201, and after aspirating the first segment of oil phase reagent 201, aspirating the magnetic bead suspension 200 to be processed.

Specifically, the aspiration operation can be performed using a syringe pump or a pressure pump.

In an embodiment, the segmented aspiration method further includes: after aspirating the magnetic bead suspension 200 to be processed, aspirating a second segment of oil phase reagent 202.

In an embodiment, a material of the flexible tube 20 may be polytetrafluoroethylene (PTFE) or the like.

Reference is made to FIGs. 9 and 10, the aspiration port end of the flexible tube 20 is hermetically connected to the sample inlet of the magnetic bead monodispersion chip, ensuring that there are no air bubbles at the connection.

Herein, ensuring that there are no air bubbles at the connection facilitates improving the monodispersity of magnetic beads and the uniformity of micro-droplet sizes.

Reference is made to FIGs. 9 and 10, after the hermetic connecting, the magnetic bead suspension 200 to be processed and the first segment of oil phase reagent 201 from the flexible tube 20 are injected into the magnetic bead monodispersion chip.

Herein, the segmented aspiration method allows, after the sample loading of the magnetic bead suspension is completed, the continued injection of the first segment of oil phase reagent into the magnetic bead monodispersion chip to push all of the magnetic bead suspension in the droplet generation array 103 into the droplet storage chamber 102. This facilitates improving the utilization rate of magnetic beads and achieving a magnetic bead utilization rate close to 100%.

The effect of magnetic bead monodispersion in the embodiment will be described below.

FIGs. 11 to 13 are schematic diagrams of magnetic bead droplets obtained by an operation method for the magnetic bead monodispersion chip according to an embodiment of the present disclosure.

Specifically, the following conditions are taken as an example:
The obtained values of the setting parameters of the magnetic bead monodispersion chip include: the width of the narrow channel region I is 25µm, the angle α is 19°, the length L of the flared region II is 150µm, the first height h of the channel is 10µm, and the height difference H is 90µm.

The surfactant used for monodispersion was Tween 20 at a concentration of 0.1%, and resuspension is performed using vortexing.

The segmented aspiration method includes: causing the flexible tube 20 to aspirate a first segment of oil phase reagent 201, after aspirating the first segment of oil phase reagent 201, aspirating the magnetic bead suspension 200 to be processed, and after aspirating the magnetic bead suspension 200 to be processed, aspirating a second segment of oil phase reagent 202.

To control the droplet size to be less than 51.8µm, the magnetic bead suspension having a volume concentration of 10,000 beads/µl is used.

Under the above conditions, the obtained magnetic bead monodispersion effect is shown in FIGs. 11 to 13.

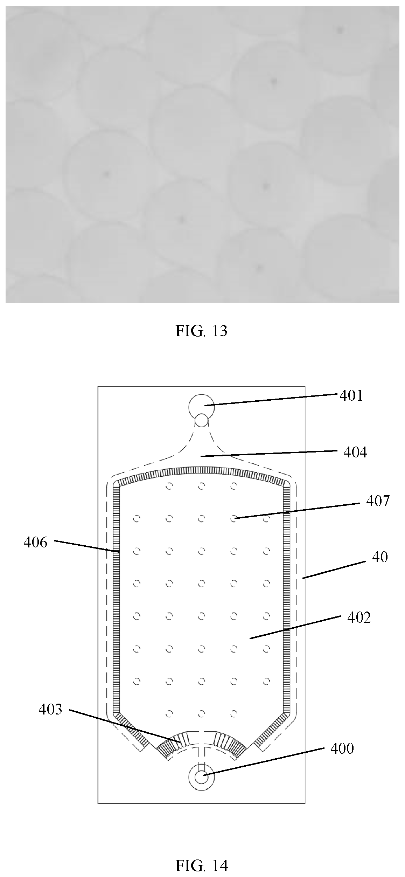

Reference is made to FIGs. 11 to 13. FIG. 11 is a schematic diagram of magnetic bead droplets. FIG. 12 is a partially enlarged view of a region A in FIG. 11. FIG. 13 is a partially enlarged view of a region B in FIG. 11. The obtained magnetic bead droplet conditions are that: the droplet size is approximately 48 µm with a CV of 1.2%, the proportion of single-bead encapsulation within the field of view is close to 100%, and only one magnetic bead retains in the generation channel, accounting for nearly 0% of the total number of magnetic beads introduced.

FIG. 14 is a schematic structural diagram of a magnetic bead monodispersion chip according to another embodiment of the present disclosure.

The main differences between the embodiment and the previous embodiment are that: the shape of the droplet storage chamber is different, and the distribution positions of the multiple support pillars are different.

In an embodiment, the droplet storage chamber is rectangular, and there are no support pillars between the bottom surface of the oil storage chamber and the upper housing.

Reference is made to FIG. 14, which is a schematic top view of the magnetic bead monodispersion chip with the upper housing omitted. The magnetic bead monodispersion chip includes: a housing, where the housing includes an upper housing (not shown in the figures) and a lower housing 40, the upper housing is provided with a sample inlet (not shown in the figures) and a sample outlet (not shown in the figures) each communicating an interior and an exterior of the housing, the sample inlet is for injecting a magnetic bead suspension into the housing, the magnetic bead suspension includes multiple to-be-processed magnetic beads, and the to-be-processed magnetic beads have a first particle size; a droplet storage chamber 402 located within the housing, where the droplet storage chamber 402 has a storage chamber bottom surface (not shown in the figures) and a storage chamber sidewall (not shown in the figures) perpendicular to the storage chamber bottom surface; a droplet generation array 403 located within the housing, where the droplet generation array 403 is further located between the droplet storage chamber 402 and the sample inlet, the droplet generation array 403 includes multiple micro-droplet generation channels, each of the channels includes a narrow channel region (not shown in the figures) and a flared region (not shown in the figures) communicating with the narrow channel region, the channel has a channel bottom surface (not shown in the figures) and a channel sidewall (not shown in the figures) perpendicular to the channel bottom surface, the channel bottom surface is higher than the storage chamber bottom surface, the narrow channel region is communicated with the sample inlet, the flared region is communicated with the droplet storage chamber 402, in a first direction perpendicular to the channel bottom surface 403d, the channel has a first height, and a ratio of the first height of the channel to the first particle size ranges from 1.5 to 3, in a second direction parallel to the channel bottom surface and perpendicular to an extension direction of the channel, the flared region has a first width adjacent to the narrow channel region and a second width adjacent to the droplet storage chamber 402, and the second width is greater than the first width; an oil storage chamber 404 located within the housing, where the oil storage chamber 404 is further located between the droplet storage chamber 402 and the sample outlet, is communicated with the sample outlet, and is for storing an oil phase reagent flowing out of the droplet storage chamber 402.

Specifically, the magnetic bead monodispersion chip further includes: a sample inlet chamber 400 located within the housing, where the sample inlet chamber 400 is located between the sample inlet and the droplet generation array 403, and is communicated with the sample inlet and the droplet generation array 403; an oil drainage chamber 401 located within the housing, where the oil drainage chamber 401 is located between the sample outlet and the oil storage chamber 404, and is communicated with the sample outlet and the oil storage chamber 404; a droplet filtering structure 406 located between the droplet storage chamber 402 and the oil storage chamber 404, where the droplet filtering structure 406 has multiple gaps (not shown in the figures) communicating the droplet storage chamber 402 and the oil storage chamber 404, a width of each of the gaps is smaller than a target droplet size; multiple support pillars 407 located between the storage chamber bottom surface and the upper housing.

Although the present disclosure is disclosed as above, the present disclosure is not limited thereto. Any person skilled in the art may make various changes and modifications without departing from the spirit and scope of the present disclosure. Therefore, the protection scope of the present disclosure should be defined by the claims.

## Claims

1. A magnetic bead monodispersion chip, comprising:
a housing, wherein the housing is provided with a sample inlet and a sample outlet each communicating an interior and an exterior of the housing, the sample inlet is for injecting a magnetic bead suspension into the housing, the magnetic bead suspension comprises a plurality of to-be-processed magnetic beads, and the to-be-processed magnetic beads have a first particle size;
a droplet storage chamber located within the housing, wherein the droplet storage chamber has a storage chamber bottom surface and a storage chamber sidewall perpendicular to the storage chamber bottom surface;
a droplet generation array located within the housing, wherein the droplet generation array is further located between the droplet storage chamber and the sample inlet, the droplet generation array comprises a plurality of micro-droplet generation channels, each of the plurality of micro-droplet generation channels comprises a narrow channel region and a flared region communicating with the narrow channel region, the micro-droplet generation channel has a channel bottom surface and a channel sidewall perpendicular to the channel bottom surface, the channel bottom surface is higher than the storage chamber bottom surface, the narrow channel region is communicated with the sample inlet, the flared region is communicated with the droplet storage chamber, in a first direction perpendicular to the channel bottom surface, the micro-droplet generation channel has a first height, a ratio of the first height of the micro-droplet generation channel to the first particle size ranges from 1.5 to 3, and in a second direction parallel to the channel bottom surface and perpendicular to an extension direction of the micro-droplet generation channel, the flared region has a first width adjacent to the narrow channel region and a second width adjacent to the droplet storage chamber, and the second width is greater than the first width; and
an oil storage chamber located within the housing, wherein the oil storage chamber is further located between the droplet storage chamber and the sample outlet, is communicated with the sample outlet, and is for storing an oil phase reagent flowing out of the droplet storage chamber.

2. The magnetic bead monodispersion chip according to claim 1, wherein a structure of the magnetic bead monodispersion chip further comprises: a droplet filtering structure located between the droplet storage chamber and the oil storage chamber, the droplet filtering structure has a plurality of gaps communicating the droplet storage chamber and the oil storage chamber, a width of each of the gaps is smaller than a target droplet size, and the width of each of the gaps ranges from 10 µm to 40 µm.

3. The magnetic bead monodispersion chip according to claim 1, wherein each of the micro-droplet generation channels further comprises an inlet region, two ends of the inlet region are communicated with the narrow channel region and the sample inlet, respectively; the inlet region has a third width adjacent to the narrow channel region and a fourth width adjacent to the sample inlet, and the fourth width is greater than the third width.

4. The magnetic bead monodispersion chip according to claim 1, wherein the droplet generation array is in a shape of a sector ring, and the plurality of micro-droplet generation channels are arranged along a circumferential direction.

5. The magnetic bead monodispersion chip according to claim 1, wherein a number of the plurality of micro-droplet generation channels is one of 8, 16, 24, 32, and 48.

6. The magnetic bead monodispersion chip according to claim 1, wherein a structure of the magnetic bead monodispersion chip further satisfies that: the first height ranges from 5 µm to 20 µm; a ratio of a width of the narrow channel region in the second direction to the first particle size ranges from 2 to 10; the width of the narrow channel region in the second direction ranges from 10 µm to 25 µm; an angle between the channel sidewall of the flared region and the channel sidewall of the narrow channel region ranges from 10 degrees to 20 degrees; a length of the flared region in the extension direction of the micro-droplet generation channel ranges from 50µm to 500µm; and a ratio of a height difference between the channel bottom surface and the storage chamber bottom surface to the first height ranges from 4 to 15.

7. The magnetic bead monodispersion chip according to claim 1, wherein the housing comprises an upper housing and a lower housing, the upper housing and the lower housing are sealed at edges, and the sample inlet and the sample outlet are located on the upper housing; the magnetic bead monodispersion chip further comprises: a plurality of support pillars located between the storage chamber bottom surface and the upper housing; the plurality of support pillars are further located between a bottom surface of the oil storage chamber and the upper housing; a radius of each of the support pillars ranges from 100µm to 500µm; the plurality of support pillars are arranged in an array in a direction parallel to the storage chamber bottom surface, a spacing distance between two adjacent support pillars ranges from 1mm to 2.5mm; a thickness of the upper housing ranges from 0.5mm to 2mm; and a thickness of the lower housing ranges from 0.5mm to 2mm.

8. The magnetic bead monodispersion chip according to claim 1, wherein a material of the magnetic bead monodispersion chip comprises one or more selected from the group consisting of glass, silicon wafer, and polymer material; the polymer material comprises one or more selected from the group consisting of polydimethylsiloxane, polyurethane, epoxy resin, polymethyl methacrylate, polycarbonate, cyclic olefin copolymer, polystyrene, polyethylene, and fluoroplastic.

9. An operation method for a magnetic bead monodispersion chip, comprising:
providing a magnetic bead monodispersion chip, wherein the magnetic bead monodispersion chip comprises:
a housing, wherein the housing is provided with a sample inlet and a sample outlet each communicating an interior and an exterior of the housing, the sample inlet is for injecting a magnetic bead suspension into the housing, the magnetic bead suspension comprises a plurality of to-be-processed magnetic beads, and the to-be-processed magnetic beads have a first particle size;
a droplet storage chamber located within the housing, wherein the droplet storage chamber has a storage chamber bottom surface and a storage chamber sidewall perpendicular to the storage chamber bottom surface;
a droplet generation array located within the housing, wherein the droplet generation array is further located between the droplet storage chamber and the sample inlet, the droplet generation array comprises a plurality of micro-droplet generation channels, each of the plurality of micro-droplet generation channels comprises a narrow channel region and a flared region communicating with the narrow channel region, the micro-droplet generation channel has a channel bottom surface and a channel sidewall perpendicular to the channel bottom surface, the channel bottom surface is higher than the storage chamber bottom surface, the narrow channel region is communicated with the sample inlet, the flared region is communicated with the droplet storage chamber, in a first direction perpendicular to the channel bottom surface, the micro-droplet generation channel has a first height, and a ratio of the first height of the micro-droplet generation channel to the first particle size ranges from 1.5 to 3, and in a second direction parallel to the channel bottom surface and perpendicular to an extension direction of the micro-droplet generation channel, the flared region has a first width adjacent to the narrow channel region and a second width adjacent to the droplet storage chamber, and the second width is greater than the first width; and
an oil storage chamber located within the housing, wherein the oil storage chamber is further located between the droplet storage chamber and the sample outlet, is communicated with the sample outlet, and is for storing an oil phase reagent flowing out of the droplet storage chamber;
providing the magnetic bead suspension;
injecting the oil phase reagent into the magnetic bead monodispersion chip through the sample inlet until no gas remains inside the magnetic bead monodispersion chip; and
after injecting the oil phase reagent, injecting the magnetic bead suspension into the magnetic bead monodispersion chip.

10. The operation method for the magnetic bead monodispersion chip according to claim 9, wherein the method of injecting the magnetic bead suspension into the magnetic bead monodispersion chip comprises:
using a segmented aspiration method to pre-encapsulate the magnetic bead suspension to be processed into a flexible tube, wherein the segmented aspiration method comprises causing the flexible tube to aspirate at least a first segment of the oil phase reagent, and after aspirating the first segment of the oil phase reagent, aspirating the magnetic bead suspension to be processed;
hermetically connecting an aspiration port end of the flexible tube to the sample inlet of the magnetic bead monodispersion chip, ensuring that there are no air bubbles at a connection; and
after hermetically connecting, injecting the magnetic bead suspension to be processed and the first segment of the oil phase reagent from the flexible tube into the magnetic bead monodispersion chip.

11. The operation method for the magnetic bead monodispersion chip according to claim 10, wherein the segmented aspiration method further comprises: after aspirating the magnetic bead suspension to be processed, aspirating a second segment of the oil phase reagent.

12. The operation method for the magnetic bead monodispersion chip according to claim 9, wherein the method of obtaining the magnetic bead suspension comprises: obtaining a target magnetic bead concentration according to a target droplet size; producing the magnetic bead suspension having the target magnetic bead concentration; or
wherein the structure of the magnetic bead monodispersion chip further comprises: a droplet filtering structure located between the droplet storage chamber and the oil storage chamber, the droplet filtering structure has a plurality of gaps communicating the droplet storage chamber and the oil storage chamber, a width of each of the gaps is smaller than a target droplet size, and the width of each of the gaps ranges from 10 µm to 40 µm; or
wherein the droplet generation array is in a shape of a sector ring, and the plurality of micro-droplet generation channels are arranged along a circumferential direction; or
wherein the housing comprises an upper housing and a lower housing, the upper housing and the lower housing are sealed at edges, and the sample inlet and the sample outlet are located on the upper housing; the magnetic bead monodispersion chip further comprises: a plurality of support pillars located between the storage chamber bottom surface and the upper housing; the plurality of support pillars are further located between a bottom surface of the oil storage chamber and the upper housing; a radius of each of the support pillars ranges from 100µm to 500µm; the plurality of support pillars are arranged in an array in a direction parallel to the storage chamber bottom surface, a spacing distance between two adjacent support pillars ranges from 1mm to 2.5mm; a thickness of the upper housing ranges from 0.5mm to 2mm; and a thickness of the lower housing ranges from 0.5mm to 2mm..

13. The operation method for the magnetic bead monodispersion chip according to claim 12, wherein the method of obtaining the target magnetic bead concentration according to the target droplet size comprises: obtaining a target droplet size D1; according to the target droplet size D1, obtaining a boundary particle size D2 of the magnetic bead suspension to be processed, wherein D2 > D1/90%; according to a relationship between a magnetic bead concentration C in the magnetic bead suspension and the boundary particle size D2, which is $\frac{1}{C} = \frac{4}{3} π {\left(\frac{D 2}{2}\right)}^{3}$, and based on the boundary particle size D2, obtaining the target magnetic bead concentration C of the magnetic bead suspension; or
wherein the method of producing the magnetic bead suspension having the target magnetic bead concentration further comprises:
obtaining an initial suspension, wherein the initial suspension comprises the plurality of to-be-processed magnetic beads;
adding a surfactant to the initial suspension, and performing a resuspension treatment on the initial suspension to obtain the magnetic bead suspension, wherein the resuspension treatment method comprises one or more of vortexing, stirring, and shaking; and the surfactant comprises one or more selected from the group consisting of Tween 20, SDS, Span 80, Triton, and EM90.

14. The operation method for the magnetic bead monodispersion chip according to claim 9, wherein the structure of the magnetic bead monodispersion chip satisfies that: the first height ranges from 5 µm to 20 µm; a ratio of a width of the narrow channel region in the second direction to the first particle size ranges from 2 to 10; the width of the narrow channel region in the second direction ranges from 10 µm to 25 µm; an angle between the channel sidewall of the flared region and the channel sidewall of the narrow channel region ranges from 10 degrees to 20 degrees; a length of the flared region in the extension direction of the micro-droplet generation channel ranges from 50µm to 500µm; and a ratio of a height difference between the channel bottom surface and the storage chamber bottom surface to the first height ranges from 4 to 15.

15. The operation method for the magnetic bead monodispersion chip according to claim 14, wherein the method of obtaining the magnetic bead monodispersion chip comprises:
establishing a model of droplet size and setting parameters of the magnetic bead monodispersion chip, wherein the setting parameters of the magnetic bead monodispersion chip comprises one or more of the first height, the width of the narrow channel region in the second direction, the first width and the second width of the flared region, an angle of inclination of a sidewall of the flared region relative to the sidewall of the narrow channel region, a length of the flared region in the extension direction of the micro-droplet generation channel, and a height difference between the channel bottom surface and the storage chamber bottom surface;
obtaining values of the setting parameters of the magnetic bead monodispersion chip according to the target droplet size; and
producing the magnetic bead monodispersion chip according to the values of the setting parameters of the magnetic bead monodispersion chip.
